# EUROPEAN PATENT APPLICATION

(11) **EP 4 374 856 A1**
(43) Date of publication of application: **29.05.2024**
(21) Application number: 23211305.0
(22) Date of filing: 21.11.2023
(51) Int. Cl.: A61K 9/20, A61K 9/28, A61K 31/496

(54) **A PROCESS FOR TABLETS COMPRISING BREXPIPRAZOLE**

(30) Priority: 23.11.2022 TR 202201770
(71) Applicant: SANOVEL ILAC SANAYI VE TICARET A.S., Istanbul 34460 (TR)
(72) Inventor: SUNEL, Fatih, Istanbul (TR); BILGEHAN ATAK, Fadime, Istanbul (TR); ATAMAN, Seval, Istanbul (TR); YAZICI, Ozlem, Istanbul (TR); SARP, Onder, Istanbul (TR)
(74) Representative: Sevinç, Erkan

(57) **Abstract**

The present invention relates to a process for the preparation of a film coated tablet comprises micronized brexpiprazole or a salt thereof as an active ingredient and at least one pharmaceutically acceptable excipient.

## Description

### Field of the Invention

The present invention relates to a process for the preparation of a film coated tablet comprises micronized brexpiprazole or a salt thereof as an active ingredient and at least one pharmaceutically acceptable excipient.

### Background of the Invention

Brexpiprazole acts as a partial agonist of the serotonin 5-HT1A receptor and the dopamine D2 and D3 receptors. Brexpiprazole has been described for use in the treatment of schizophrenia, depression and other central nervous system disorders, and for use in the prophylaxis and/or treatment of behavioral and psychological symptoms associated with neurodegenerative disease or impulsive symptoms associated with mental disease.

Brexpiprazole is a compound represented by the following Formula I, and its chemical name is 7-[4-[4-(1-benzothiophen-4-yl)piperazin-1-yl]butoxy]-1H-quinolin-2-one.

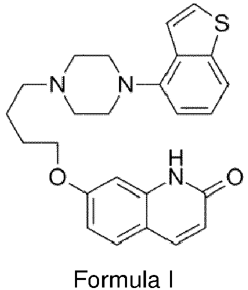

Brexpiprazole is an antidepressant and antipsychotic drug marketed under the brand Rexulti^{®} for the oral treatment of schizophrenia and as an adjunctive treatment to antidepressants in major depressive disorder. REXULTI tablets are intended for oral administration and available in 0.25 mg, 0.5 mg, 1 mg, 2 mg, 3 mg and 4 mg strengths. The product was approved in the U.S. in 2015 for the aforementioned indications and is currently in phase III trials for the treatment of agitation associated with Alzheimer's disease and the treatment of PTSD (post-traumatic stress disorder).

Brexpiprazole has poor solubility in water. The major limitation of the drug is its low solubility and permeability leading to poor bioavailability. So, in a formulation comprising brexpiprazole, solubility is important.

WO 2013/054872 patent application discloses pharmaceutical formulations comprising brexpiprazole and methods for their manufacture. In particular, the document describes producing uncoated or coated tablets of brexpiprazole using the following components: brexpiprazole, lactose, corn starch, microcrystalline cellulose, low-substituted hydroxypropylcellulose, hydroxypropylcellulose and magnesium stearate. The compositions described therein are prepared by wet granulation techniques.

CN105412036 patent application discloses an orally disintegrating tablet containing Brexpiprazole where the improvement in dissolution is provided by the reduction of the active substance particle size below 10 pm in a course of co- grinding process utilizing lactose.

In prior art, low solubility of brexpiprazole has been approached in several ways. However, these studies in the prior art have been it strongly affects the dissolution profile of pharmaceutical compositions comprising brexpiprazole, and therefore causes problems with the development of a pharmaceutical formulation with a specified dissolution profile. it is not the case that only the careful selection of excipients, binders, disintegrants and other ingredients customarily used in pharmaceutical compositions or reducing particle size of active agent is important for the development of a composition with favorable solubility characteristics.

There is thus still a need for a process for the preparation of a film coated tablet comprises micronized brexpiprazole having the desired stability, dissolution profile, hardness and compressibility, in another words the disadvantages seen in the active substance will be able to overcome.

### Detailed Description of the Invention

The main object of the present invention is to eliminate problems caused by active substance, brexpiprazole, and bringing additional advantages to the relevant prior art.

Another object of the present invention is to provide a film coated tablet comprising brexpiprazole with high stability, having desired level of dissolution rate which overcomes the above described problems in prior art and have additive advantages over them.

Another object of the present invention is to provide a film coated tablet comprising brexpiprazole having improved content uniformity and homogeneity and compressibility.

As used here in the present invention, "micronized brexpiprazole" means brexpiprazole has a d (0.9) particle size is less than 40 µm. The cumulative volume size distrubition is tested by any conventionally accepted method such as the laser diffraction method (i.e. Malvern Mastersizer 2000 analysis).

In one embodiment of the invention, brexpiprazole or a salt thereof has a d (0.9) particle size between 35 µm and 1 µm, preferably between 20 µm and 1 µm.

We have found that brexpiprazole or a salt thereof having the following particle sizes is very important for formulation. Especially, it positively affects the dissolution properties and powder homogenization. The obtained film coated tablets have the desired dissolution profile. The powder is more homogeneous. The content uniformity of the film coated tablets obtained from the more homogeneous powder is more ideal. Therefore, it provides better bioavailability. So, in the present invention, micronized brexpiprazole is used.

The ratio of Brexpiprazole in the tablet weight is too low and micronized form is used, that may result in agglomerations during powder blending and content non-uniformity. Especially, for this reason, we used the binder in the preparation of the granulation solution. We saw that the use of a binder in the preparation of granulation solution surprisingly achieved the desired stability, content uniformity, hardness, flowability and homogeneity.

According to an embodiment of the present invention, the film coated tablet is prepared wet granulation. Wet granulation process efficiently counteracts the segregation of active agent, so it is observed that the desired stability, tablet hardness, content uniformity and compressibility.

So, an easy method is created to eliminate the disadvantages of active ingredient and also the method is simple and cost-effective method. Also, this process helps to provide the desired dissolution profile.

According to one embodiment of the present invention, a process for the preparation of a film coated tablet comprises micronized brexpiprazole or a salt thereof comprising the following steps:
a) Mixing micronized brexpiprazole, at least one filler and at least one disintegrant,
b) Dissolving at least one binder in water,
c) Granulating the powder mixture at step (a) with the mixture at step (b).

According to one embodiment of the present invention, the amount of brexpiprazole or a salt thereof is between 0.1% and 10.0% by weight of the total composition.

In general terms, excipients provided in a formulation may positively or negatively influence the physicochemical and pharmacokinetic properties, e.g. the solubility, absorption, bioavailability of an active agent. For this reason, the excipients which accompany an active agent have to be selected in a careful and conscious manner while a formulation is developed. The formulations should have no physicochemical incompatibility between the active agent and the excipients.

According to one embodiment of the invention, at least one binder is dissolved in a solvent and a binder solution is obtained. For this reason, it ensures that active agents and excipients adhere to each other in an ideal way and provides homogeneous granule units. In this way, possible blend uniformity and content uniformity problems that may be seen in the product are prevented.

Suitable binders are selected from the group comprising polyvinylpyrrolidone, carboxymethylcellulose sodium, sugars, agar, alginates, carbomers, cellulose acetate phthalate, chitosan, starch, corn starch, dextrose, ethylcellulose, glyceryl behenate, hydrogenated vegetable oil type I, hydroxyethyl cellulose, hydroxyethyl methyl cellulose, hydroxypropyl cellulose, hydroxypropyl starch, hypromellose, magnesium aluminum silicate, maltodextrin, methylcellulose, poloxamer, polycarbophil, polydextrose, polyethylene oxide, polymethacrylates, sucrose, polyoxyethilene-alkyl ethers, pullulan or mixtures thereof.

According to this embodiment of the present invention, the binder is polyvinylpyrrolidone.

According to this embodiment of the present invention, the amount of binder in the tablet is 0.1% to 20.0% by weight of the total composition.

According to this embodiment of the present invention, the amount of binder in the tablet is 0.1% to 10.0% by weight of the total composition.

Suitable fillers are selected from the group comprising lactose monohydrate, microcrystalline cellulose, mannitol, pregelatinized starch, ammonium alginate, calcium carbonate, anhydrous lactose, calcium phosphate, calcium phosphate dehydrate, neutral pellets, calcium sulfate, cellulose, cellulose acetate, dextrates, dextrin, dextrose, erythritol, fructose, glyceryl palmitostearate, magnesium carbonate, magnesium oxide, maltodextrin, maltose, medium chain triglycerides, polydextrose, polymethacrylates, sodium alginate, sodium chloride, sorbitol, starch, sucrose, sugar sphericals, sulfobutylether beta-cyclodextrin, talc, tragacanth, trehalose, polysorbate 80, xylitol or mixtures thereof.

According to this embodiment of the present invention, the filler is lactose monohydrate, microcrystalline cellulose or mixtures thereof.

According to this embodiment of the present invention, the amount of filler in the tablet is 60.0% to 90.0% by weight of the total composition.

Suitable disintegrants are selected from the group comprising sodium starch glycolate, crospovidone, cross-linked carboxymethyl cellulose (croscarmellose sodium), low-substituted hydroxypropyl cellulose, carboxymethyl cellulose, docusate sodium, guar gum, sodium alginate, corn starch, alginic acid, magnesium aluminium silica, poloxamer, sodium glycine carbonate or mixtures thereof.

According to this embodiment of the present invention, the disintegrant is sodium starch glycolate. Especially, using sodium starch glycolate helps to provide the desired dissolution profile. Sodium starch glycolate absorbs water rapidly, resulting in swelling which leads to rapid disintegration of tablets.

According to this embodiment of the present invention, the amount of disintegrant in the tablet is 0.1% to 20.0% by weight, preferably 0.1% to 10.0% by weight of the total composition.

According to one embodiment of the present invention, a process for the preparation of a film coated tablet comprises micronized brexpiprazole or a salt thereof comprising the following steps:
a) Mixing brexpiprazole, lactose monohydrate, microcrystalline cellulose and sodium starch glycolate for 15 minutes,
b) Dissolving polyvinylpyrrolidone in water,
c) Granulating the powder mixture at step (a) with the mixture at step (b).

According to this embodiment of the present invention, the film coated tablet further comprises at least one lubricant.

Suitable lubricants are selected from the group comprising sodium stearyl fumarate, magnesium stearate, calcium stearate, zinc stearate, talc, boric acid, hydrogenated vegetable oil, sodium chlorate, magnesium lauryl sulfate, sodium oleate, sodium acetate, sodium benzoate, polyethylene glycol, stearic acid, fatty acid, fumaric acid, glyceryl palmito sulphate, sodium lauryl sulphate or mixtures thereof.

According to this embodiment of the present invention, the lubricant is sodium stearyl fumarate. It improves the powder processing properties of film coated tablet formulation. Also, it enhances powder flow by reducing the inter-particle friction.

According to this embodiment of the present invention, the amount of lubricant in the tablet is 0.1% to 5.0% by weight of the total composition.

In another embodiment of this present invention, a film coat on the tablet protects from moisture and further contributes to the ease with which it can be swallowed. Also, it is used in order to minimize for stability problems.

According to this embodiment of the present invention, the film coated tablet comprises;
▪ Brexpiprazole or a salt thereof having particle size d90 less than about 40 microns,
▪ 0.1-25.0% by weight of sodium starch glycolate
▪ 0.1 -5.0% by weight of lubricants of the total composition.

According to this embodiment of the present invention, the film coated tablet comprises;
▪ Brexpiprazole or a salt thereof having particle size d90 less than about 40 microns,
▪ Sodium starch glycolate
▪ Sodium stearyl fumarate

According to one embodiment of the present invention, a process for the preparation of a film coated tablet comprises micronized brexpiprazole or a salt thereof comprising the following steps:
a) Mixing brexpiprazole, lactose monohydrate, microcrystalline cellulose and sodium starch glycolate for 15 minutes,
b) Dissolving polyvinylpyrrolidone in water,
c) Granulating the powder mixture at step (a) with the mixture at step (b),
d) Sieving the wet granules and then drying,
e) Sieving the dried granules and adding sodium stearyl fumarate and then mixing for 3 minutes,
f) Compressing the total mixture into tablets
g) Coating the tablets with film coating.

### Example 1: The film coated tablet is prepared by wet granulation with water

| **Ingredients** | **Amount (% by weight of the total formulation)** |
|---|---|
| Brexpiprazole | 0.1 -10.0 |
| Lactose monohydrate | 40.0 - 60.0 |
| Microcrystalline cellulose | 20.0 - 50.0 |
| Sodium starch glycolate | 0.1 -10.0 |
| Polyvinylpyrrolidone K-30 | 0.1 -10.0 |
| Sodium stearyl fumarate | 0.1 - 5.0 |
| Water | q.s. |
| **TOTAL** | **100** |
| **Film coating** | |

### Example 2: The film coated tablet is prepared by wet granulation with water

| **Ingredients** | **Amount (% by weight of the total formulation)** |
|---|---|
| Brexpiprazole | 0.52 |
| Lactose monohydrate | 53.32 |
| Microcrystalline cellulose | 38.16 |
| Sodium starch glycolate | 4 |
| Polyvinylpyrrolidone K-30 | 3 |
| Sodium stearyl fumarate | 1 |
| Water | q.s. |
| **TOTAL** | **100** |
| **Film coating** | |

### Example 3: The film coated tablet is prepared by wet granulation with water

| **Ingredients** | **Amount (% by weight of the total formulation)** |
|---|---|
| Brexpiprazole | 4.4 |
| Lactose monohydrate | 49.4 |
| Microcrystalline cellulose | 38.16 |
| Sodium starch glycolate | 4 |
| Polyvinylpyrrolidone K-30 | 3 |
| Sodium stearyl fumarate | 1 |
| Water | q.s. |
| **TOTAL** | **100** |
| **Film coating** | |

A process for example 1 or 2 or 3;
a) Mixing brexpiprazole, lactose monohydrate, microcrystalline cellulose and sodium starch glycolate for 15 minutes,
b) Dissolving polyvinylpyrrolidone in water,
c) Granulating the powder mixture at step (a) with the mixture at step (b),
d) Sieving the wet granules and then drying,
e) Sieving the dried granules and adding sodium stearyl fumarate and then mixing for 3 minutes,
f) Compressing the total mixture into tablets
g) Coating the tablets with film coating.

### Film coating materials;

| |
|---|
| Hypromellose, |
| Talc |
| Titanium Dioxide |
| Iron oxide red or yellow or black |

## Claims

1. A process for the preparation of a film coated tablet comprises micronized brexpiprazole or a salt thereof comprising the following steps:
a) Mixing micronized brexpiprazole, at least one filler and at least one disintegrant,
b) Dissolving at least one binder in water,
c) Granulating the powder mixture at step (a) with the mixture at step (b).

2. A process according to claim 1, wherein the amount of brexpiprazole or a salt thereof is between 0.1% and 10.0% by weight of the total composition.

3. A process according to claim 1, wherein binders are selected from the group comprising polyvinylpyrrolidone, carboxymethylcellulose sodium, sugars, agar, alginates, carbomers, cellulose acetate phthalate, chitosan, starch, corn starch, dextrose, ethylcellulose, glyceryl behenate, hydrogenated vegetable oil type I, hydroxyethyl cellulose, hydroxyethyl methyl cellulose, hydroxypropyl cellulose, hydroxypropyl starch, hypromellose, magnesium aluminum silicate, maltodextrin, methylcellulose, poloxamer, polycarbophil, polydextrose, polyethylene oxide, polymethacrylates, sucrose, polyoxyethilene-alkyl ethers, pullulan or mixtures thereof.

4. A process according to claim 3, wherein the binder is polyvinylpyrrolidone.

5. A process according to claim 1, wherein fillers are selected from the group comprising lactose monohydrate, microcrystalline cellulose, mannitol, pregelatinized starch, ammonium alginate, calcium carbonate, anhydrous lactose, calcium phosphate, calcium phosphate dehydrate, neutral pellets, calcium sulfate, cellulose, cellulose acetate, dextrates, dextrin, dextrose, erythritol, fructose, glyceryl palmitostearate, magnesium carbonate, magnesium oxide, maltodextrin, maltose, medium chain triglycerides, polydextrose, polymethacrylates, sodium alginate, sodium chloride, sorbitol, starch, sucrose, sugar sphericals, sulfobutylether beta-cyclodextrin, talc, tragacanth, trehalose, polysorbate 80, xylitol or mixtures thereof.

6. A process according to claim 5, wherein the filler is lactose monohydrate, microcrystalline cellulose or mixtures thereof.

7. A process according to claim 1, wherein disintegrants are selected from the group comprising sodium starch glycolate, crospovidone, cross-linked carboxymethyl cellulose (croscarmellose sodium), low-substituted hydroxypropyl cellulose, carboxymethyl cellulose, docusate sodium, guar gum, sodium alginate, corn starch, alginic acid, magnesium aluminium silica, poloxamer, sodium glycine carbonate or mixtures thereof.

8. A process according to claim 7, wherein the disintegrant is sodium starch glycolate.

9. A process according to claim 1, wherein comprising the following steps:
a) Mixing brexpiprazole, lactose monohydrate, microcrystalline cellulose and sodium starch glycolate,
b) Dissolving polyvinylpyrrolidone in water,
c) Granulating the powder mixture at step (a) with the mixture at step (b).

10. A process according to claim 1, wherein film coated tablet further comprises at least one lubricant.

11. A process according to claim 10, wherein lubricants are selected from the group comprising sodium stearyl fumarate, magnesium stearate, calcium stearate, zinc stearate, talc, boric acid, hydrogenated vegetable oil, sodium chlorate, magnesium lauryl sulfate, sodium oleate, sodium acetate, sodium benzoate, polyethylene glycol, stearic acid, fatty acid, fumaric acid, glyceryl palmito sulphate, sodium lauryl sulphate or mixtures thereof.

12. A process according to claim 10, wherein the lubricant is sodium stearyl fumarate.

13. A process according to claim 1, wherein comprising the following steps:
a) Mixing brexpiprazole, lactose monohydrate, microcrystalline cellulose and sodium starch glycolate,
b) Dissolving polyvinylpyrrolidone in water,
c) Granulating the powder mixture at step (a) with the mixture at step (b),
d) Sieving the wet granules and then drying,
e) Sieving the dried granules and adding sodium stearyl fumarate and then mixing,
f) Compressing the total mixture into tablets
g) Coating the tablets with film coating.
